# EUROPEAN PATENT SPECIFICATION

(11) **EP 1 907 412 B1**
(45) Date of publication and mention of the grant of the patent: **11.04.2012**
(21) Application number: 06757830.2
(22) Date of filing: 29.06.2006
(51) Int. Cl.: C07K 5/062, C07K 5/068, C07K 5/083, C07K 5/09, C07K 5/103, C07K 5/11, C07K 5/117, C12P 21/06, A23J 3/34, A61K 38/01, A61K 38/04, A61K 38/17

(54) **PEPTIDES INHIBITING ANGIOTENSIN-CONVERTING ENZYME**
DAS ANGIOTENSIN-CONVERTING ENZYME INHIBIERENDE PEPTIDE
PEPTIDES AYANT UNE ACTIVITÉ INHIBANTE DE ACE

(30) Priority: 30.06.2005 EP 05105977
(43) Date of publication of application: 09.04.2008
(73) Proprietor: Campina Nederland Holding B.V., 5301 LB Zaltbommel (NL)
(72) Inventor: CADEE, Jenneke, Adriana, NL-3984 KA Odijk (NL); MALLEE, Leon, Franciscus, NL-5971 GE Grubbenvorst (NL)
(74) Representative: Swinkels, Bart Willem
(86) International application number: PCT/NL2006/050155
(87) International publication number: WO 2007/004876

(56) References cited:
- WO-A-02/19837
- WO-A-2004/104182
- WO-A-2006/068480
- WO-A1-2006/108211
- WO-A1-2006/125441
- US-B1- 6 514 941
- HERNÁNDEZ-LEDESMA BLANCA ET AL: "Angiotensin converting enzyme inhibitory activity in commercial fermented products. Formation of peptides under simulated gastrointestinal digestion." JOURNAL OF AGRICULTURAL AND FOOD CHEMISTRY. 24 MAR 2004, vol. 52, no. 6, 24 March 2004 (2004-03-24), pages 1504-1510, XP008057959 ISSN: 0021-8561
- DATABASE WPI Derwent Publications Ltd., London, GB; AN 1996-515010 XP002438148 & JP 08 269087 A (YAMAUCHI F) 15 October 1996 (1996-10-15)
- BULAJ G ET AL: "Denaturation of free and complexed bovine trypsinogen with the calcium ion, dipeptide Ile-Val and basic pancreatic trypsin inhibitor (Kunitz)." EUROPEAN JOURNAL OF BIOCHEMISTRY / FEBS. 1 AUG 1994, vol. 223, no. 3, 1 August 1994 (1994-08-01), pages 939-946, XP002361483 ISSN: 0014-2956
- VAN ELSWIJK D A ET AL: "Rapid detection and identification of angiotensin-converting enzyme inhibitors by on-line liquid chromatography-biochemical detection, coupled to electrospray mass spectrometry" JOURNAL OF CHROMATOGRAPHY, ELSEVIER SCIENCE PUBLISHERS B.V. AMSTERDAM, NL, vol. 1020, no. 1, 5 December 2003 (2003-12-05), pages 45-58, XP004687429 ISSN: 0021-9673 cited in the application
- PHARM.RES., vol. 14, no. 10, 1997, pages 1332-1340,
- DATABASE WPI Section Ch, Week 199408, Derwent Publications Ltd., London, GB; Class B04, AN 1994-061999 & JP 06 016568 A (CHIBA SEIHUN) 25 January 1994
- MATSUI E.A.: JOURNAL OF PEPTIDE SCIENCE, vol. 5, 1999, pages 289-297,
- TOKUNAGA E.A.: BIOL.PHARM.BULL., vol. 27, no. 2, 2004, pages 189-192,
- LEHTINEN E.A.: "In vitro effects of bioactive tripeptides, Ile-Pro-Pro and Val-Pro-Pro on ACE and Chymase", PROCEEDINGS 4TH INT.PEPTIDE SYMPOSIUM, 2007, pages 1-2,

## Description

The invention pertains to novel peptides that are capable of inhibiting angiotensin-converting enzyme (ACE) and to their use in the control and prevention of hypertensia.

### Background

Angiotensin-converting enzyme (ACE) converts angiotensin I to angiotensin II, which causes contraction of vascular smooth muscle cells. In addition, ACE deactivates the vasodilator bradykinin. Hence, ACE has a hypertensive effect. Agents capable of inhibiting ACE are thus potential hypotensives. Several peptides are known to have an ACE-inhibiting (ACEI) capacity, some of which are derived from milk-proteins.

US 5,449,661 (Nakamura et al.) discloses the production of the ACEI tripeptide VPP by fermentation of milk by a *Lactobacillus helveticus* strain JCM 1004. WO 01/032836 and WO 01/032905 (Mäyrä-Mäkinen et al.) describe the production of VPP and IPP by fermentation with *L. helveticus* strain LBK-16H (DSM 13137). WO 01/034828 (Yamamoto et al.; = EP-A 1 231 279) describes the production of VPP and IPP by a two-step enzymatic proteolysis of casein.

US 6,514,941 (Tolton et al.) describes three peptides having ACEI effect, obtained by trypsin hydrolysis of acid casein. These are denominated C7 (AVPYPQR) (derived from β-casein), and C12 (FFVAPFPEVFGK) and C6 (TTMPLW) (both derived from α-S2 casein).

EP-A 821 968 (Yamamoto) describes the production of the ACEI dipeptide YP by fermentation of milk by a *Lactobacillus helveticus* strain FERM BP-4835.

WO 02/071854 (Draaisma et al.) describes ACEI peptides obtained by fermentation of milk with *Lactobacillus delbrueckii lactis,* having the β-casein sequences DKIHPF and KVLPVPQ or specific parts thereof.

WO 04/002509 (Tauzin et al.) describes ACEI peptides obtained by trypsin hydrolysis of α-S2 casein, having the sequences TVY, NMAINP(SK), FALP(QY(LK)), YL, FPQYLQY and ALNEINQFY(QK).

WO 02/019837 (Schlothauer et al.) describes hydrolysis of whey proteins using specific proteases yielding peptides SAP, MKG, ALPMH, LIVTQ, LKPTPEGDLEIL, INYWL and (LKGYGG)VSLPEW.

Van Elswijk et al., J. Chromatography A, 1020 (2003) 45-58, describe the detection and identification of ACEI peptides from milk proteins, and report bioactivity of peptides WLAHK, ALPMHIR, GLDIQK, FALPQYLK, FFVAPFPEVFGK (C12), AVPYPQR (C7), and other ones. WO 04/098310 describes IIAEK, IPAVF and IPAVK as ACE inhibiting peptides from β-lactoglobulin.

WO 04/104182 describes bioactive peptides obtained by fermentation of milk with specific strains of *Enterococcus faecalis.* The peptides include the β-casein-derived peptides LHLPLP, VLGPVRPFP and VVVPPF.

JP-A 6-016568 discloses tripeptides from wheat gluten having ACE inhibiting activity, including Ile-Ile-Tyr.

Matsui et al., J. Peptide Sc. 5: 289-297 (1999), describe wheat germ peptides having ACE-inhibiting activity, including Ile-Val-Tyr.

Tokunaga et al., Biol. Pharm. Bull. 27, 189-192 (2004) reports that peptides Ile-Tyr, Val-Tyr and Ile-Val-Tyr from Royal Jelly ihibit ACE. activity.

WO 2006/125441, published 30-11-2006, describes dipeptides Leu-Val and Ile-Val. According to Table 1, these dipeptides have ACE inhibitor effect which is significantly better than Ile-Pro-Pro.

### Description of the invention

*Novel peptides having ACE-inhibiting activity were found according to the* invention. These novel peptides can be obtained by enzymatic proteolysis of milk fractions, milk proteins or other proteins.

In one embodiment, the invention provides peptides having ACE-inhibiting activity for use in the treatment or prevention of hypertension the peptides being selected from the following sequences AIV, IIV, LIV and IVP.

The invention also concerns peptide mixtures for use in the treatment or prevention of hypertension. In particular, such a peptide mixture contains, on a peptide basis, at least 1 wt.%, preferably at least 3 wt.% especially at least 5 wt.%, of the individual peptides IV, LV, AVP.

A preferred peptide mixture according to the invention contains AVP, LV and IV. The preferred amounts of these peptides in the relevant mixture are those as indicated above, i.e. at least 1 wt.% for the individual peptide, and at least 5 wt.% for the particular combination, on a peptide basis.

A particular group of ACE-inhibiting peptides is disclosed, which are di-and tripeptides containing two adjacent aliphatic amino acids, the N-terminal one of which is selected from Ala, Val, Ile and Leu, and the C-terminal one is selected from Val, Ile and Leu. The optional third (C-terminal) amino acid is preferably one of the neutral small amino acids Gly, Ala and Pro, and more preferably one of the smallest amino acids Gly and Ala, if the N-terminal amino acid is a large amino acid Val, Ile or Leu. Examples thereof include IV, LV, AVP, and AIV.

The peptides of the invention can be prepared by specific hydrolysis of milk proteins or other protein source. This can be done using proteolytic enzymes, either as such, or by fermentation with a microorganism containing such enzyme in an active form. Combinations of enzymes and/or microorganisms, either sequential or as a mixture, can also be used. Suitable enzymes include commercially available proteases, such as trypsin, chymotrypsin, Alcalase (Novozymes), Neutrase (Novozymes) Protease N, Protease P6, Protease A (Amano), Flavourzyme (Novozymes), Promod 25P (Biocatalysts, UK), Multifect Neutral (Genencor), Fungal protease (Bio-Cat Inc, USA), Orientase 90N (Hankyu, Japan), Enzeco Fungal Protease 100A (EDC, USA), Proteinase K, pepsin, papain, thermolysin, elastase, Arg-C proteinase, Asp-N proteinase. Furthermore, besides proteases, (exo)peptidases may be used, e.g. to reduce the bitterness of the hydrolysates obtained, or to remove specific C- or N-terminal amino acids in order to obtain the desired peptides. Examples of peptidases are e.g. leucine aminopeptidases or carboxypeptidases A, B, C and/or Y.

The enzymatic process of the invention can be carried out on milk proteins of any mammal, in particular milk proteins from ungulates, especially ruminants, more in particular members from the family of the Bovidae. The Bovidae include cattle and allies (Bovinae) and goats and allies (Caprinae). Preferred Bovinae species include cattle, yak, buffalo and water buffalo; preferred Caprinae species include sheep and goat. Most preferably the milk protein is bovine milk protein.

In a preferred embodiment, the enzyme reaction is carried out by :
1) contacting the protein source with a first protease, the protease comprising a neutral protease;
2) optionally contacting the protein source a second protease or peptidase, or contacting the protein source with a mixture of said first protease and said second protease or peptidase;
3) inactivating and/or separating the proteases and optionally further fractionating and./or purifying the hydrolysate obtained.

The first protease may be a bacterial (e.g. from *Bacillus* spp.) or fungal neutral protease. Preferably this protease may be selected from the group Neutrase, Protease P6, Protease A, Promod 25P, Multifect Neutral and Fungal Protease.

The second protease or peptidase may be an exoprotease or an endoprotease, or a mixture of the two. Preferred examples of these enzymes include Flavourzyme, Orientase 90N and Enzeco Fungal Protease 100A.

The first protease and the second protease or peptidase may be applied simultaneously or one after the other, with or without intermediate inactivation of the first protease. For example, the hydrolysis may be carried out using the first protease until a degree of hydrolysis of e.g. 3-10 %, followed by optional inactivation, and addition of the second protease or peptidase, and further hydrolysis, until the desired total degree of hydrolysis.

The enzymatic reaction is carried out using the conditions which are appropriate for the particular enzyme or enzyme combination. The reaction conditions thus may comprise temperatures between 20 and 60°C, depending on the optimum temperature of the enzymes, pH values e.g. between 3 and 10, depending on the optimum pH of the enzymes, and reaction times that may vary between about 30 min. and 24 h. The reaction times are selected depending on the reaction temperature, and on the concentration, activity and specificity of the enzymes. Generally, reaction conditions are such that a relatively high level of short peptides (less than 12 amino acids) is formed. Degrees of hydrolysis thus may vary, e.g. between 3 and 30 %, especially between about 5 and 20 %, or even above 7.5 or more preferably at least 10 %. When the desired degree of hydrolysis is attained, the hydrolysis reaction can be terminated by separation of the proteolytic enzyme (e.g. in the case of immobilised enzymes), or by inactivation of the enzymes by methods depending on the individual protease, such as acidification to pH 3-4.5, or heating to between 70 and 120°C for 1 sec to 30 minutes, especially for 5 sec. to 5 min. at between 80 and 100°C.

It was found that the proteolysis medium affects the proteolysis results and the composition of the hydrolysate. Firstly, the level of cations, especially alkali metal and alkaline earth metals, can advantageously be controlled in such a manner that the level of cations from the third period, especially sodium and magnesium, is relatively low, and the level of cations from the fourth period, especially potassium, and calcium, is relatively high.

Thus, during proteolysis, the combined concentration of K and Ca is preferably larger than the combined concentration ofNa and Mg, preferably (K+Ca)/(Na+Mg) is at least 2:1 and more preferably between 3: 1 and 9:1. Preferably the K + Ca level is between 10 en 500 mM (400 mg - 20 g/l), more preferably 15 - 400 mM, even more preferably 20 - 300 mM, most preferably 40 - 200, and especially 50 - 100 mM. The individual concentrations are preferably 5 - 400, more preferably 10 - 200 and most preferably 25 - 100 mM for K and preferably 5 - 300, more preferably 10 - 150 and most preferably 20 - 75 mM for Ca. Concentrations of Na and Mg are preferably below 30 mM (700 mg/l) and below 20 mM (480 mg/l), respectively.

The presence of the alkali metal and alkaline earth metal cations can be controlled by choosing the appropriate starting material and/or by adding the metals before or during hydrolysis. For example, when preparing casein-derived peptides, it is advantageous to use calcium caseinate as a starting material rather than sodium caseinate. Also, when adjusting the pH before or during hydrolysis, this is preferably done by adding potassium hydroxide or other potassium of calcium-containing salts (e.g. phosphate, citrate, lactate, (bi)carbonate, gluconate), rather than sodium hydroxide or sodium salts.

An additional advantage of the use of potassium and calcium instead of sodium is, that the final peptide or peptide preparation contains a relatively low amount of sodium, which is generally seen as favourable regarding the treatment of hypertension.

Also, during the hydrolysis reaction, a water-miscible cosolvent may be used in addition to water. The amount of cosolvent is preferably between 0.5 and 30 wt %, more preferably between 1 and 20 wt. %, most preferably between 2 and 10 wt. % of the hydrolysis medium. Suitable cosolvents include acetone, alkanols like methanol, ethanol, n-propanol or i-propanol; dimethyl sulfoxide (DMSO); dimethylformamide (DMF); or ethers such as dioxane. The cosolvents may be used alone or in combination with each other.

Another useful hydrolysis method is described in US 6,514,941, e.g. using trypsin as the proteolytic enzyme until an average molar weight below 5000 Da is achieved, followed by ultrafiltration and diafiltration. This procedure can be followed e.g. by Sepharose chromatography.

The hydrolysates obtained can be subjected to further separation using filtration (ultra, nano), chromatography (affinity, ion exchange or other), electrophoresis, extraction, precipitation, and other techniques known in the art, and combinations of such techniques. The final hydrolysate product may be "polished" by filtering over a filter aid, e.g. diatomaceous earth, to remove any insoluble matter.

The individual peptides may also be prepared by chemical synthesis.

When a mixture of active peptides is desired, e.g. skim milk, can be the starting material. The milk may be bovine, goat, sheep, horse, buffalo, yak, human, preferably bovine milk. It is preferred for practical reasons to start with a milk fraction or with a processed, e.g. ultrafiltrated milk, such the salt concentration is reduced. When single peptides are desired, it may be advantageous to start from isolated milk proteins, e.g. isolated or mixed caseinates, whey protein isolates, whey protein concentrates, or isolated whey proteins such as α-lactalbumin or β-lactoglobulin or bovine serum albumin or fractions or partial hydrolysates thereof. When casein is used as a starting material, the serine residues may also be phosphorylated.

Other protein sources are e.g. soy (e.g. yielding an active peptide LR), wheat (including gluten), cottonseed, lupin, pea, potato and other suitable plant proteins; other animal proteins, e.g. egg, blood, meat, fish (bonito, tuna).

It may also be advantageous to perform a post-treatment of the hydrolysates, so as to further diversify or activate the peptide mixture or for debittering the mixture. Such a post-treatment can for example be fermentation with suitable microorganisms, such as lactic acid bacteria, *Streptococcus thermophilus, Lactobacillus delbrueckii, Lb. delbrueckii* subsp. *bulgaricus, Lb. acidophilus" Lb. casei, Lb. helveticus* and *Bifidobacterium bifidum,* or combinations thereof. An example of a suitable treatment comprises fermenting with a yoghurt culture (*S. thermophilus* and *Lb. d. bulgaricus*). Such a fermentation can be performed on the hydrolysate as such, or on a combination with milk or a milk product. Also other nutrients, such as peptone, tryptone, etc., may be present. The treatment van be performed using conventional conditions for such fermentations, although shorter fermentation periods can also be applied. The ACE inhibiting activity of the product may increase or decrease, but should not decrease with more than 50%, preferably no more than 20% with respect to the starting hydrolysate.

The invention also concerns a food product as defined in claim 3 i.e. containing at least one of the peptides together with one or more of lactic acid bacteria, especially the bacteria mentioned above.

The identity and the activity of the peptides and their mixtures can be determined by known methods, e.g. as described by Van Elswijk et al. J. Chromatography A, 1020 (2003) 45-58, involving liquid chromatography coupled to mass spectroscopy, and on-line bioassay. The active peptides of the invention are characterised by exhibiting an ACE inhibiting concentration (IC₅₀) of below 1500 µM, preferably below 750 µM, more preferably below 250 µM or even below 100 µM. The IC₅₀ can be determined in vitro as described by V. Vermeirsen, J. van Camp & J. Verstraete, "Optimisation and validation of an angiotensin-converting enzyme inhibition assay for the screening of bioactive peptides". J. Biochem. Biophys. Methods 51, 75-87 (2002). It is noted that peptides having only moderate in vitro activity (relatively high IC₅₀) may become relatively more activity in the body as a result of further modification, e.g. splitting off of one or more amino acids.

The invention also pertains to milk protein hydrolysates that are enriched in the ACEI peptides described above. These hydrolysates contain at least 1 wt.% (on dry weight basis) of one or more of the active peptides as defined herein, up to e.g. 50 wt.%. Preferably, the hydrolysates contain between 2 and 40 wt.%, more preferably between 5 and 25 wt.% of the peptides as defined herein. The remainder may be non-active peptides and proteins and/or other active peptides, as well as minor amounts (e.g. below 25 wt.%, preferably between 0.1 and 10 wt.%) of non-proteinaceous material.

The peptides of the invention, as well as their mixtures or mixtures with other peptides are suitable for application in foods, more specifically in functional foods or supplements for the treatment or prevention of hypertension. Functional foods comprise milk or fermented milk products, like yoghurt, beverages, nutrition bars and the like. Optionally, stabilizers (pectin, carragheenan), emulsifiers (mono- and diglycerides), thickeners (alginic acid, chitosan or salts thereof) can be included in the food product. The concentration of the peptides in the food product may be such that between 5 and 500 mg is present per 100 g dry food, or per litre of liquid food. Suitable supplements are e.g. tablets and powders for instant drinks.

The peptides or a specific peptide mixture of the invention may also be suitable for pharmaceutical application to treat or prevent hypertension. The peptides can be administered at a level of between 1 - 500 mg active peptide/peptide mixture per individual per day. Preferred dosage levels are between 10 and 150 mg/day. In terms of the hydrolysates of the invention containing between 1 and 50 wt/.% of active peptides, these can be administered at a level of between 2 mg and 10 g, depending i.a. on the concentration of active peptides. Preferred dosage levels of the hydrolysates are between 10 mg and 5 g, more preferably between 50 mg and 1 g.

A pharmaceutical composition can comprise the daily dosage level in one dosage unit or in multiple (e.g. 2-6) daily dosage units). The pharmaceutical composition can further contain conventional excipients, stabilisers, flavours, colorants and the like. In particular, the composition may contain an anionic or other polysaccharides as an excipient, for example carboxymethylcellulose, pectin, chitosan, agar, or, especially alginic acid. The anionic polysaccharide may also be in the salt form The weight ratio between the alginic acid or other polysaccharide and the active peptide may e.g. be between 4:1 and 50:1, especially between 9:1 and 24:1. The pharmaceutical composition may have the form of tablets, capsules, coated tablets, lozenges, syrups, powders, sachets, solutions, suspensions etc. The total weight of the pharmaceutical composition may be in the range of 100 - 1000 mg of a dry form, e.g. 400-800 mg in case of a tablet.

### Examples

The examples are comparative as far as not relating to peptides of the invention.

### Example I: Preparation of a protein hydrolysate containing ACE inhibiting peptides.

Calcium caseinate (DMV International, NL) (2.7 kg) was dissolved in water (16 litres), at a temperature of approx. 50° C. An amount of ethanol was added (0.6 litre 50 wt %), and the pH was adjusted to 7.2 using KOH (45 wt%). The total amount of Ca and K was about 90 mM. Neutral Fungal protease from *Aspergillus oryzae* (Bio-Cat, Inc.) was added in an amount of 0.2 % (w/w on protein), and subsequently Flavourzyme (0.1 % w/w), and hydrolysis proceeded for 10 hours at 50°C. To terminate the enzyme reaction, a heating step was carried out (10 minutes 90° C). The solution was then filtered over diatomaceous earth to remove any insoluble particles, and dried to obtain the hydrolysate powder.

The degree of hydrolysis of the product is expressed as the AN/TN ratio (amino nitrogen/total nitrogen)* 100. The AN/TN ratio was 12.4. Amino nitrogen is determined according to the Formol titration method. Total nitrogen can be determined using the Kjeldahl method.

### Example II: Preparation of a protein hydrolysate containing ACE inhibiting peptides.

A 12 % (w/w) solution of WPC 80 (ArlaFoods, Denmark) in water was made, and the pH was adjusted to 7.0 using a 45 wt % KOH solution. An amount of 0.35 % Multifect Neutral (bacterial neutral protease, Genencor) was added and the hydrolysis was continued for 6 hours at 50° C. Then the pH was re-adjusted to 6.7 using KOH. The amount of Ca + K was approximately 45 mM.

The reaction mixture was heated to 85°C for 30 seconds to inactivate the enzyme. The solution was concentrated by evaporation to 20 % solids and spray-dried. The AN/TN ratio of the product was 10.

### Example III_{:} Identification of ACE-inhibiting peptides.

The peptides obtained according to example I, were analysed using HPLC as described by Van Elswijk et al. J. Chromatography A 1020 (2003) 45-58. The HPLC eluate was divided over a mass spectrometry for determining the peptide structure and a bioassay unit for assessing the ACE-inhibiting activity. The following peptides were identified: AVP, LV and IV. Another batch of the same caseinate contained the peptides KIHP, KK, VPYPQR, and KYKVPQ. Starting with other casein sources the peptides VLGPV, VLPVPQK, VPYPQ, KK, VNELSK, and FFVAPFPEVFG were found.

Similarly the product from example II was analysed and contained IAEKT, LDIQK and LKALP. Starting from other whey protein sources WPC 60 or whey protein isolate, VLGPV, VLPVPQ, MKGLDI, LR, MHIRLS, IIA were identified.

### Example IV: Antihypertensive composition (tablets)

| Ingredients | weight % |
|---|---|
| Hydrolysate of example I | 98.8 |
| Magnesium stearate NF FG D | 0.5 |
| (Crompton Corporation) | |
| Stearic Acid Hystrene 5016 NF FG V | 0.5 |
| (Crompton Corporation) | |
| Silicon dioxide Cab-O-Sil M-5 | 0.2 |
| (DMV International GmbH) | |

### Preparation:

The hydrolysate is thoroughly mixed with the silicon dioxide. Magnesium stearate and stearic acid are added, and mixed for 1 minute. Tablets are prepared by direct compression. The tablets are coated with ChromaTone P DDB8876-BL, Chr. Hansen.

### Tableting parameters:

| | |
|---|---|
| Weight: | 840 mg |
| Length (oblong): | 19 mm |
| Thickness: | 6 mm |
| Compression force: | 18.7 kN |
| Hardness: | 180 N |
| Disintegration time: | 11.5 minutes |

### Example V_{:} Antihypertensive food

| Ingredients | Weight % |
|---|---|
| Skimmed milk | 78.0 |
| Water | 11.0 |
| Sugar | 7.0 |
| Maltitol (C*Maltidex 16385, Cerestar) | 3.0 |
| Hydrolysate example II | 0.7 |
| Pectin (Genupectin YM115-L CP Kelco) | 0.3 |
| Pineapple flavour (B80583, Takasago, Japan) | 0.03 |
| Peach flavor (B 80361, Takasago, Japan) | 0.04 |
| Colour (Riboflavine 503065, | 0.001 |
| Sensient Food Colours) | |
| Citrosa (Flavor, NHDC, Exquim S.A., Spain) | 0.0003 |
| Lactic acid | q.s. |
| Culture ABT-2 (Chr. Hansen) | q.s. |

### Preparation:

A stock solution (4%) of pectin in (part of the) water is prepared at 70°C. The milk and the remaining water is mixed, the hydrolysate, sugar, maltitol and citrosa are dissolved in the milk, and the pH is adjusted to 6.7 using lactic acid.

After pasteurisation at 90 °C for 5 minutes, the mixture is cooled to fermentation temperature (37°C), and the culture is added. Fermentation is continued until the pH is 4.2. The pH is then adjusted to 4.0 using lactic acid. The pectin solution is added, while mixing vigorously. After homogenisation at 120/20 bar at 40°C, the flavours and colour are added and the product is filled into bottles and stored cool.

## Claims

1. A peptide having ACE-inhibiting activity for use in the treatment or prevention of hypertension, the peptide being selected from AIV, IIV, LIV and IVP.

2. A peptide mixture for use in the treatment or prevention of hypertension, the mixture containing at least the peptides IV, LV and AVP.

3. A nutritional composition containing one or more peptides according to claim 1, or a mixture according to claim 2, together with at least one nutritional ingredient for use in the treatment or prevention of hypertension.

4. A pharmaceutical composition containing one or more peptides according to claim 1, or a mixture according to claim 2, together with at least one pharmaceutically acceptable carrier for use in the treatment or prevention of hypertension.

## Patentansprüche

1. Peptid mit ACE-inhibierender Wirkung zur Verwendung für die Behandlung oder die Prävention von Hypertension, wobei das Peptid aus AIV, IIV, LIV und IVP ausgewählt ist.

2. Mischung von Peptiden zur Verwendung für die Behandlung oder die Prävention von Hypertension, wobei die Mischung zumindest die Peptide IV, LV und AVP enthält.

3. Nahrungsmittel das ein oder mehrere Peptide nach Anspruch 1, oder eine Mischung nach Anspruch 2, zusammen mit mindestens einem Nahrungsbestandteil enthält, zur Verwendung für die Behandlung oder die Prävention von Hypertension.

4. Pharmazeutische Zusammensetzung die ein oder mehrere Peptide nach Anspruch 1, oder eine Mischung nach Anspruch 2, zusammen mit mindestens einem Pharmazeutisch annehmbaren Träger enthält, zur Verwendung für die Behandlung oder die Prävention von Hypertension.

## Revendications

1. Peptide ayant une activité inhibitrice de l'ECA pour utilisation dans le traitement ou la prévention de l'hypertension, le peptide étant sélectionné parmi AIV, IIV, LIV et IVP.

2. Mélange de peptides pour utilisation dans le traitement ou la prévention de l'hypertension, le mélange contenant au moins les peptides IV, LV et AVP.

3. Composition nutritionnelle contenant un ou plusieurs peptides selon la revendication 1, ou un mélange selon la revendication 2, avec au moins un ingrédient nutritionnel, pour utilisation dans le traitement ou la prévention de l'hypertension.

4. Composition pharmaceutique contenant un ou plusieurs peptides selon la revendication 1, ou un mélange selon la revendication 2, avec au moins un support pharmaceutiquement acceptable, pour utilisation dans le traitement ou la prévention de l'hypertension.
